# EUROPÄISCHE PATENTANMELDUNG

(11) **EP 2 711 702 A1**
(43) Veröffentlichungstag der Anmeldung: **26.03.2014**
(21) Anmeldenummer: 12185788.2
(22) Anmeldetag: 25.09.2012
(51) Int. Cl.: G01N 33/28

(54) **Verfahren zur Ermittlung eines Alterungszustands eines Schmierstoffs**

(71) Anmelder: Siemens Aktiengesellschaft, 80333 München (DE)
(72) Erfinder: Rohde, Joachim, 52070 Aachen (DE)

(57) **Zusammenfassung**

Die Erfindung betrifft ein Verfahren zur Ermittlung eines Alterungszustands eines Schmierstoffs (6), der in einer zu schmierenden Maschine (5) mit einer metallischen Oberfläche (7) in Kontakt kommt, sowie eine Vorrichtung (1) zur Durchführung des Verfahrens. Das Verfahren umfasst eine Bestimmung eines Gehalts an Metall-Ionen bzw. einer Änderung eines Gehalts an Metall-Ionen im Schmierstoff (6).

## Beschreibung

Die vorliegende Erfindung betrifft ein Verfahren zur Ermittlung eines Alterungszustands eines Schmierstoffs, sowie eine Vorrichtung zur Ausführung des Verfahrens.

Die Schmierstoffqualität ist im Maschinen- und Anlagenbau und insbesondere in der Getriebetechnik eine wesentliche Einflussgröße, die die Verfügbarkeit, die Zuverlässigkeit und die Sicherheit des gesamten Antriebsstranges oder der geschmierten Baukomponente bestimmt. Die Erfahrung in der Wartung von Getrieben zeigt, dass auch die besten Schmierstoffe altern und gewechselt werden müssen. Dabei geht man immer mehr von zeitgeplanten Wechselintervallen zu zustandsabhängigen Wechselfristen über.

Allgemein gültige Kriterien, wann die Qualität eines Schmierstoffs unzureichend wird, existieren jedoch noch nicht. In der Fachliteratur gibt es eine große Anzahl von Wechselkriterien, die einander zum Teil widersprechen. In der Regel ist für jede Anwendung und jeden Schmierstoff ein individueller Grenzwert zu beachten.

Der Erfindung liegt die Aufgabe zugrunde, ein verbessertes Verfahren zur Ermittlung eines Alterungszustands eines Schmierstoffs bereit zu stellen, sowie eine Vorrichtung zur Ausführung des Verfahrens.

Diese Aufgabe wird erfindungsgemäß durch ein Verfahren mit den in Anspruch 1 angegebenen Merkmalen gelöst.

Das erfindungsgemäße Verfahren dient einer Ermittlung eines Alterungszustands eines Schmierstoffs, der in einer zu schmierenden Maschine mit einer metallischen Oberfläche in Kontakt kommt. Das Verfahren umfasst eine Bestimmung eines Gehalts an Metall-Ionen bzw. einer Änderung eines Gehalts an Metall-Ionen im Schmierstoff.

Der Begriff Maschine umfasst allgemein alle bewegten Maschinenteile, in denen Schmierstoff zur Schmierung verwendet wird, z.B. Getriebe, insbesondere Zahnradgetriebe, und Motoren. Ein Schmierstoff kann ein flüssiger Schmierstoff, z.B. ein Schmieröl, aber auch ein pastöser Schmierstoff, z.B. ein Fließfett oder Schmierfett, sein.

Der Erfindung liegt die Erkenntnis zugrunde, dass ein alternder Schmierstoff aggressive Anteile bildet, welche eine metallische Oberfläche chemisch angreifen können, und dass ein dadurch hervorgerufenes Herauslösen von Metall-Ionen aus der metallischen Oberfläche als ein Indikator für einen Alterungszustand eines Schmierstoffs genutzt werden kann.

Da ein Großteil der Bauteile in geschmierten Maschinen in der Regel aus Eisen bzw. Stahl besteht, ist der Nachweis von Eisen-Ionen im Schmiermittel bevorzugt. Es können aber auch Ionen anderer Metalle, z.B. Kupfer oder Zinn, als Nachweis für den Alterungszustand des Schmiermittels dienen. Die Metallart der Ionen kann einen Hinweis darauf geben, welche Bauteile von dem Schmierstoff angegriffen werden. Eisenionen können auf einen Angriff von Zahnrädern und Wälzlagern hinweisen. Kupferionen können auf einen Angriff von Messingkäfigen und Messing- oder Bronzelagern hinweisen. Zinnionen können auf einen Angriff von Gleitlagern aus Weißmetall oder Bronze hinweisen.

Solange der Schmierstoff die Metalloberfläche nicht angreift, werden keine bzw. relativ wenig Metall-Ionen aus der Oberfläche herausgelöst. Werden Metall-Ionen herausgelöst, muss man prüfen, ob dies eine Folge eines gewollten bzw. geduldeten Prozesses, z.B. eine Reaktion eines dem Schmierstoff hinzugefügten Additivs, oder ein unerwünschter, stark beschleunigter Austrag von Metall-Ionen aufgrund von Schmierstoffalterung ist.

Die vorliegende Erfindung ermöglicht eine zuverlässige Überwachung der Qualität eines in einer Maschine befindlichen Schmierstoffs. Der Vorteil des vorliegenden Verfahrens gegenüber bekannten Verfahren zur Bestimmung einer Schmierstoffalterung wie z.B. einer Messung einer Säurezahl oder einer Auswertung eines Infrarot-Spektrums liegt vor allem in einer einfachen Festlegung eines Grenzwertes. Ein Herauslösen von Metall-Ionen aus einer metallischen Oberfläche findet erst dann beschleunigt statt, wenn die Aggressivität des Schmierstoffs zunimmt. Zur Festlegung eines Grenzwertes bedarf es somit keiner aufwändigen Versuche, welche jeweils nur spezifisch für individuelle Kombinationen von Anwendung und Schmierstoff gelten. Stattdessen kann für jeden Schmierstoff ein Grenzwert mittels einfacher Laborprüfungen festgelegt werden.

Vorteilhafte Ausgestaltungen und Weiterbildungen der Erfindung sind in den abhängigen Ansprüchen angegeben. Dabei kann das erfindungsgemäße Verfahren auch entsprechend den abhängigen Vorrichtungsansprüchen weitergebildet sein, und umgekehrt.

Gemäß einer bevorzugten Ausgestaltung der Erfindung erfolgt die Bestimmung durch einen Messsensor, welcher in der Maschine angeordnet ist oder über eine Schmierstoffleitung damit verbunden ist. Der Schmierstoffzustand kann direkt im Betrieb der zu schmierenden Maschine mithilfe eines Sensors erfolgen, der Metall-Ionen detektiert.

Gemäß einer bevorzugten Ausgestaltung der Erfindung erfolgt die Bestimmung durch eine Entnahme einer Schmierstoffprobe und eine Analyse der Schmierstoffprobe. Der Schmierstoffzustand kann durch eine chemische Analyse einer Schmierstoffprobe in einem Labor erfolgen.

Eine Labormethode basierend auf Nachweisreagenz ist ein Kationennachweis für Eisenionen im Reagenzglas mittels einer Nachweisreagenz, nämlich der Eisenionennachweis mit Thiocyanat, Thioglykolsäure oder rotem bzw. gelbem Blutlaugensalz. Eine nachfolgende quantitative Bestimmung kann mittels Spektroskopie erfolgen.

Außerdem wird diese Aufgabe erfindungsgemäß durch eine Vorrichtung mit den in Anspruch 4 angegebenen Merkmalen gelöst.

Die erfindungsgemäße Vorrichtung dient einer Durchführung eines Verfahrens nach einer der oben beschriebenen Ausgestaltungen. Die Vorrichtung umfasst einen Sensor zur Bestimmung eines Gehalts an Metall-Ionen bzw. einer Änderung eines Gehalts an Metall-Ionen im Schmierstoff. Die Vorrichtung umfasst einen Prozessor zur Auswertung von im Sensor gewonnenen Messwerten. Die Vorrichtung umfasst außerdem eine Übertragungseinheit zur Übertragung der Messwerte von dem Sensor zu dem Prozessor.

Der Schmierstoffzustand kann auch durch eine physikalische Nachweismethode von Metall-Ionen erfolgen.

Gemäß einer bevorzugten Ausgestaltung der Erfindung umfasst der Sensor ionenselektive Elektroden zur Messung einer Konzentration von Metallionen.

Gemäß einer bevorzugten Ausgestaltung der Erfindung umfasst der Sensor ein photometrisches Analysesystem zur Messung von Metallionen. Derartige photometrische Analysesysteme kommen z.B. bei einer Messung von Eisenionen in Wasser oder in der Medizintechnik zum Einsatz.

Im Folgenden wird die Erfindung anhand mehrerer Ausführungsbeispiele unter Zuhilfenahme der beiliegenden Zeichnung erläutert. Es zeigt jeweils schematisch und nicht maßstabsgetreu
- Fig. 1: einen zeitlichen Verlauf einer Anzahl von Metall-Ionen in einem Schmierstoff;
- Fig. 2: ein erstes Ausführungsbeispiel einer Vorrichtung zur Durchführung des Verfahrens; und
- Fig. 3: ein zweites Ausführungsbeispiel einer Vorrichtung zur Durchführung des Verfahrens.

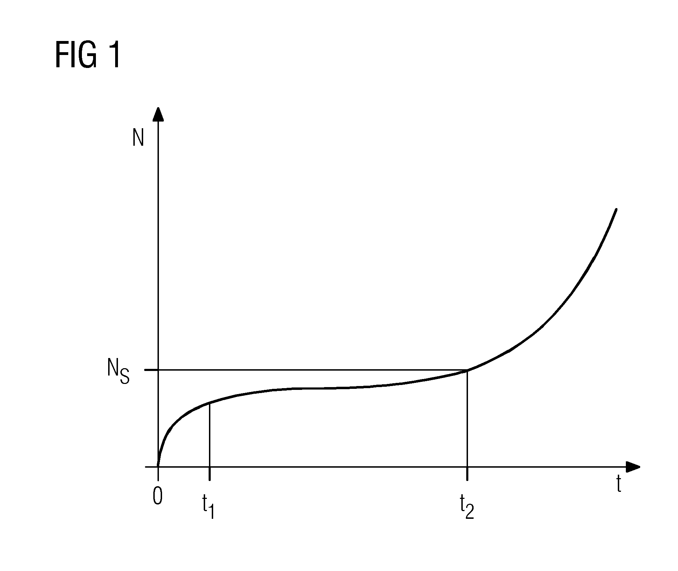

Fig. 1 zeigt einen Graph, in dem eine Anzahl von Metall-Ionen N in einem Schmierstoff, der in einer zu schmierenden Maschine mit einer metallischen Oberfläche in Kontakt kommt, über die Zeit t aufgetragen ist. Die Zeitachse beginnt beim Zeitpunkt 0, der einer Inbetriebnahme der Maschine entspricht. Bis zu einem Zeitpunkt t1 steigt die Anzahl von Metall-Ionen N im Schmierstoff stark an, was durch einen Einlauf der Maschine bedingt ist: bis sich die miteinander in Kontakt befindlichen Maschinenteile, z.B. Zahnräder und Wellen, "eingelaufen" haben, erfolgt ein relativ großer Abrieb und damit ein Übergang von Eisen-Partikeln und auch Eisen-Ionen in den Schmierstoff.

In einem Zeitintervall zwischen t1 und t2 ist der Schmierstoff noch so unverbraucht, dass er nur einen relativ geringen und nur wenig ansteigenden Gehalt an aggressiven chemischen Verbindungen aufweist, welche Eisen-Ionen aus der metallischen Oberfläche der zu schmierenden Maschine herauslösen. Daher ist in dem Zeitintervall zwischen t1 und t2 die Anzahl von Metall-Ionen N in dem Schmierstoff relativ gering.

Zu dem Zeitpunkt t2 beträgt die Zahl an Metall-Ionen N im Schmierstoff Nₛ (S = Schwellwert). Ab einem Zeitpunkt t2 steigt die Zahl an Metall-Ionen N in dem Schmierstoff stark an. Der Grund dafür ist, dass der alternde Schmierstoff eine zunehmend größer werdende Zahl an aggressiven Anteilen bildet, welche eine metallische Oberfläche chemisch angreifen können. Aufgrund der wachsenden Aggressivität des Schmierstoffs findet ein Herauslösen von Metall-Ionen aus einer metallischen Oberfläche beschleunigt statt. Der Zeitpunkt t2, zu dem definiert somit einen Zeitpunkt, zu dem der Schmierstoff ausgetauscht werden sollte. Dieser Zeitpunkt t2, zu dem die Zahl an Metall-Ionen N im Schmierstoff den Wert Nₛ annimmt, kann gemäß der vorliegenden Erfindung durch Bestimmung des Gehalts N an Metall-Ionen ermittelt werden: Wenn der Gehalt an Metall-Ionen den Wert Nₛ erreicht, ist der Schmierstoff-Wechsel-Zeitpunkt t2 erreicht.

Alternativ kann auch eine Änderung des Gehalts an Metall-Ionen im Schmierstoff 6 überwacht werden. Dabei nimmt man das Zeitintervall zwischen t=0 und t1 aus, weil die Ursache des darin auftretenden starken Anstiegs nicht der Alterungsprozess des Schmierstoffs ist. In dem Zeitintervall zwischen t1 und t2 ist die Änderung der Anzahl von Metall-Ionen N in dem Schmierstoff relativ langsam. Erst ab dem Zeitpunkt t2, wenn der Schmierstoff bereits gealtert ist, ist eine ausgeprägte Änderung der Anzahl von Metall-Ionen N in dem Schmierstoff, nämlich ein starker Anstieg, zu beobachten. Das heißt, anstatt der absoluten Anzahl an Metall-Ionen im Schmierstoff kann auch eine zeitliche Änderung der Anzahl an Metall-Ionen im Schmierstoff als Indiz für den Alterungszustand des Schmierstoffs dienen.

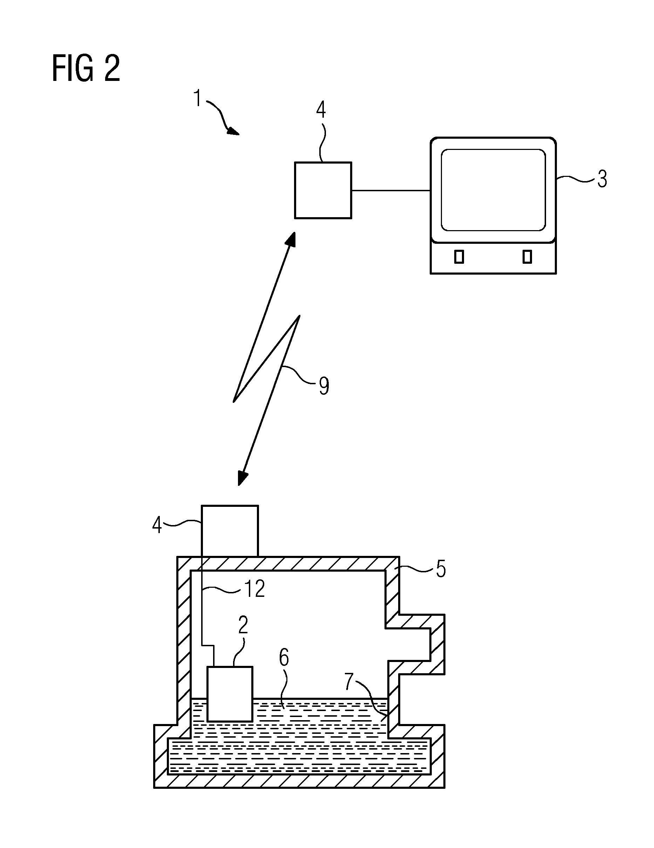

Fig. 2 zeigt eine Vorrichtung 1 zur Durchführung eines Verfahrens zur Ermittlung eines Alterungszustands eines Schmierstoffs 6, welcher in einer zu schmierenden Maschine 5 mit einer metallischen Oberfläche 7 in Kontakt kommt.

Im Inneren der Maschine 5 ist ein Sensor 2 installiert, der zur Bestimmung eines Gehalts an Metall-Ionen bzw. einer Änderung eines Gehalts an Metall-Ionen im Schmierstoff dient. Der Sensor taucht teilweise in einen mit Schmierstoff 6 gefüllten Ölsumpf ein und ermittelt Messwerte, welche eine Aussage zur Konzentration von Metall-Ionen im Schmierstoff erlauben. Beispielsweise umfasst der Sensor 2 ionenselektive Elektroden, die in Kontakt mit dem Schmierstoff sind.

Die von dem Sensor 2 gewonnenen Messwerte werden über eine Datenleitung 12 zu einer Übertragungseinheit 4 übertragen, welche eine kabellose Übertragung 9 der Messwerten zwischen einer Sendeeinheit und einer korrespondierenden Empfangeinheit ausführt. Von der Empfangseinheit werden die Messwerte schließlich zu einem Prozessor 3 übertragen, der eine Auswertung der vom Sensor 2 gewonnenen Messwerte vornimmt. Falls sich aus den Messwerten ergibt, dass der Gehalt an Metall-Ionen über einem Schwellwert Nₛ liegt, erzeugt der Prozessor 3 eine Benachrichtigung an einen Betreiber der Vorrichtung 1, z.B. als Warnhinweis auf einem Bildschirm oder als eine E-Mail.

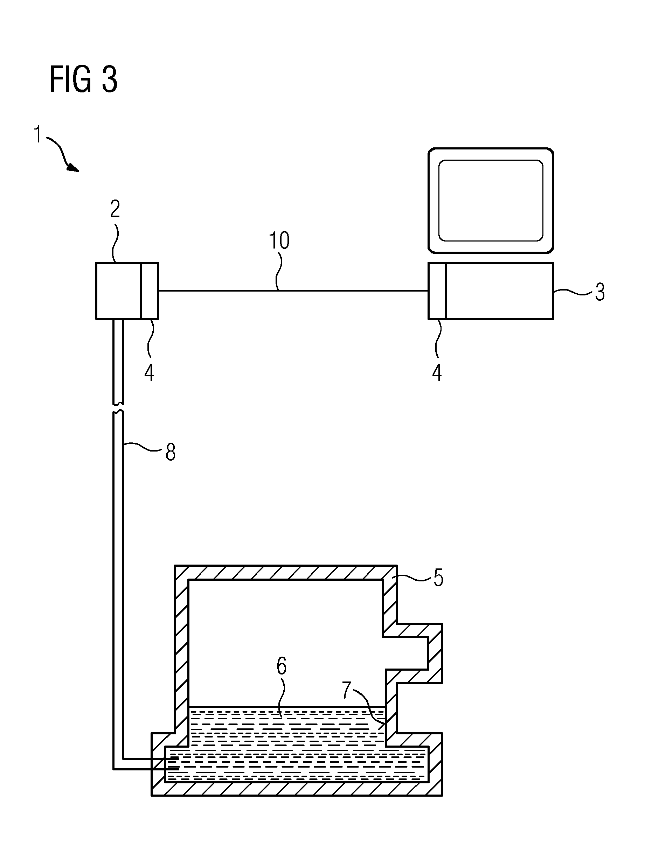

Fig. 3 zeigt ebenfalls eine Vorrichtung 1 zur Durchführung eines Verfahrens zur Ermittlung eines Alterungszustands eines Schmierstoffs 6, welcher in einer zu schmierenden Maschine 5 mit einer metallischen Oberfläche 7 in Kontakt kommt. Dabei ist der Sensor 2, der zur Bestimmung eines Gehalts an Metall-Ionen bzw. einer Änderung eines Gehalts an Metall-Ionen im Schmierstoff dient, im Gegensatz zu der in Fig. 2 dargestellten Vorrichtung nicht im Inneren der Maschine 5, sondern au-βerhalb der Maschine 5 angeordnet.

Der externe Sensor 2 ist über eine Schmierstoffleitung 8 mit einem Schmierstoffsammelbehälter, z.B. einem Ölsumpf, der Maschine 5 verbunden. Über die Schmierstoffleitung 8 wird dem Sensor Schmierstoff 6 aus der Maschine 5 zugeführt. Dies entspricht einer Entnahme einer Schmierstoffprobe. Die Schmierstoffleitung 8 ist vorzugsweise als eine Ringleitung ausgebildet, mit einem von der Maschine 5 zu dem Sensor 2 führenden Schmierstoff-Vorlauf und einem in entgegen gesetzter Richtung führenden Schmierstoff-Rücklauf. Auf diese Weise kann kontinuierlich dem Sensor 2 "frischer" Schmierstoff 6 aus der Maschine 5, d.h. Schmierstoff mit aktuellem Metall-Ionengehalt, als Probenmaterial zugeführt werden.

Der Sensor 2 ermittelt auf Basis des ihm zugeführten Schmierstoffs 6 Messwerte, welche eine Aussage zur Konzentration von Metall-Ionen im Schmierstoff 6 erlauben. Dies entspricht einer Analyse der Schmierstoffprobe. Beispielsweise umfasst der Sensor 2 ein photometrisches Analysesystem zur Messung von Metall-Ionen.

Die von dem Sensor 2 gewonnenen Messwerte werden an eine Übertragungseinheit 4 übertragen, welche via Datenkabel 10 eine Übertragung der Messwerte zwischen einer Sendeeinheit und einer korrespondierenden Empfangeinheit ausführt. Von der Empfangseinheit werden die Messwerte schließlich zu einem Prozessor 3 übertragen, der eine Auswertung der vom Sensor 2 gewonnenen Messwerte vornimmt. Falls sich aus den Messwerten ergibt, dass der Gehalt an Metall-Ionen über einem Schwellwert Nₛ liegt, erzeugt der Prozessor 3 eine Benachrichtigung an einen Betreiber der Vorrichtung 1, z.B. als Warnhinweis auf einem Bildschirm oder als eine E-Mail.

Obwohl die Erfindung im Detail durch die bevorzugten Ausführungsbeispiele näher illustriert und beschrieben wurde, so ist die Erfindung nicht durch die offenbarten Beispiele eingeschränkt, und andere Variationen können vom Fachmann hieraus abgeleitet werden, ohne den Schutzumfang der Erfindung zu verlassen.

## Patentansprüche

1. Verfahren zur Ermittlung eines Alterungszustands eines Schmierstoffs (6), der in einer zu schmierenden Maschine (5) mit einer metallischen Oberfläche (7) in Kontakt kommt, umfassend folgenden Schritt:
Bestimmen eines Gehalts an Metall-Ionen bzw. einer Änderung eines Gehalts an Metall-Ionen im Schmierstoff (6).

2. Verfahren nach Anspruch 1, wobei die Bestimmung durch einen Messsensor (2) erfolgt, welcher in der Maschine (5) angeordnet ist oder durch eine Schmierstoffleitung (8) damit verbunden ist.

3. Verfahren nach Anspruch 1 oder 2, wobei die Bestimmung durch eine Entnahme einer Schmierstoffprobe und eine Analyse der Schmierstoffprobe erfolgt.

4. Verfahren nach einem der Ansprüche 1 bis 3, umfassend folgenden Schritte:
Vergleich des bestimmten Gehalts bzw. der bestimmten Änderung mit einem Referenzwert; und
Auslösen einer Benachrichtigung, falls der Gehalt bzw. die Änderung den Referenzwert überschreitet.

5. Vorrichtung (1) zur Durchführung eines Verfahrens nach einem der Ansprüche 1 bis 4, umfassend:
- einen Sensor (2) zur Bestimmung eines Gehalts an Metall-Ionen bzw. einer Änderung eines Gehalts an Metall-Ionen im Schmierstoff;
- einen Prozessor (3) zur Auswertung von im Sensor (2) gewonnenen Messwerten; und
- eine Übertragungseinheit (4) zur Übertragung der Messwerte von dem Sensor (2) zu dem Prozessor (3).

6. Vorrichtung (1) nach Anspruch 5, wobei der Sensor (2) ionenselektive Elektroden umfasst.

7. Vorrichtung (1) nach Anspruch 5 oder 6, wobei der Sensor (2) ein photometrisches Analysesystem umfasst.
